Europäisches Patentamt

European Patent Office

Office européen des brevets

(11) Publication number: **0 142 642**
**A1**

## (12) EUROPEAN PATENT APPLICATION

(21) Application number: **84110127.2**

(22) Date of filing: **24.08.84**

(51) Int. Cl.⁴: **A 61 L 2/18**
**A 01 N 59/00, G 02 C 13/00**

(30) Priority: **25.08.83 US 526567**

(43) Date of publication of application:
**29.05.85 Bulletin 85/22**

(84) Designated Contracting States:
**AT BE CH DE FR GB IT LI LU NL SE**

(71) Applicant: **Barnes-Hind, Inc.**
**895 Kifer Road**
**Sunnyvale California(US)**

(72) Inventor: **Kasper, Hans H.**
**20822 Meadow Oak Road**
**Saratoga, Calif.(US)**

(72) Inventor: **Sibley, Murray J.**
**224 Stanford Ave**
**Berkeley, Calif.(US)**

(72) Inventor: **Chu, Victor C.**
**665 Blythe Court No. 37**
**Sunnyvale, Calif.(US)**

(74) Representative: **Brauns, Hans-Adolf, Dr. rer. nat. et al,**
**Hoffmann, Eitle & Partner, Patentanwälte**
**Arabellastrasse 4**
**D-8000 Munich 81(DE)**

(54) Process and kit for sodium sulfite neutralization of H2O2 in contact lenses.

(57) Residual $H_2O_2$ remaining on a contact lens that has been disinfected in an $H_2O_2$ solution is neutralized by placing the lens in a buffered saline solution which contains an effective amount of sodium sulfite. Provided is also a kit including means for washing the lenses and a source for sodium sulfite.

EP 0 142 642 A1

## PROCESS AND KIT FOR SODIUM SULFITE NEUTRALIZATION OF $H_2O_2$ IN CONTACT LENSES

This application relates to the disinfection of contact lenses, and more particularly to disinfection by water-borne chemical agents without the application of heat.

Contact lenses accumulate dirt, proteinaceous matter, and microorganisms, all of which can adversely affect the health of the eye if allowed to accumulate on the lens. Therefore, the lenses must be cleaned and disinfected regularly and preferably daily.

It is generally known that hydrogen peroxide, in aqueous solution at a concentration of 3 wt. %, can be used to disinfect contact lenses and simultaneously remove unwanted dirt and proteinaceous matter. However, the hydrogen peroxide (hereafter, $H_2O_2$) will irritate the eye if even a small residual amount remains on the lens when it is re-inserted into the eye. This problem is especially notable with soft contact lenses, which are made from a water-permeable polymer into which the $H_2O_2$ can penetrate. Removing the $H_2O_2$ from such material is particularly difficult, and has required extensive washing and soaking with saline solution. Thus, it is desirable to be able to employ $H_2O_2$ in disinfecting contact lenses, while avoiding the irritancy of residual $H_2O_2$.

U.S. Patent No. 3,829,329 discloses immersing soft contact lenses in a normal saline (NaCl) aqueous solution of 3% $H_2O_2$, to shrink the lenses. The lenses were then boiled for 2 hours each in distilled water and normal saline solution. Adopting such a severe boiling regimen for the daily treatment of soft contact lenses would be inconvenient to the user and could cause the lenses to deteriorate.

U.S. Patent No. 3,908,680 discloses a treatment regimen for plastic articles such as contact lenses, which employs two boiling, aqueous baths containing a peroxy compound such as $H_2O_2$, followed by cleansing with a nonionic detergent and rinsing with distilled water. This sequence, besides being overly cumbersome for daily application, is not sure to remove all residual $H_2O_2$.

U.S. Patent No. 3,912,451 discloses that $H_2O_2$ in a solution used to sterilize soft contact lenses can be subsequently neutralized by contacting the solution with a metallic catalyst which decomposes the $H_2O_2$. This system is not sure to work quickly, as it relies on diffusion of the $H_2O_2$ to a solid metallic surface. Such a system also becomes less and less effective as the concentration of $H_2O_2$ declines; unfortunately, the $H_2O_2$ concentration at which this system loses efficiency can still be high enough to risk irritation to the eye of the user. In addition, this system requires several manual or mechanical steps to bring the metallic catalyst into contact with the $H_2O_2$ solution. One such mechanical approach is disclosed in U.S. Patent No. 4,013,410, in which a mechanical timer rotates the container holding the solution so as to bring the solution into contact with a band of catalytic metal.

An article by A. R. Gasset et al., "Hydrogen Peroxide Sterilization of Hydrophilic Contact Lenses", in Arch. Ophthalmol, Vol. 93 (June, 1975) pp. 412-415, discusses sterilizing soft contact lenses in 3% $H_2O_2$ solution. A solution of sodium thiosulfate was used to neutralize the residual $H_2O_2$ remaining in the lenses after sterilization. The authors found that sodium thiosulfate concentrations of 1.5% and 2.0% were unable to destroy all residual $H_2O_2$, while a 2.5% solution was effective. However, sodium thiosulfate needs a rather lengthy reaction time to ensure that all the peroxide is neutralized.

The present invention provides a convenient, safe, quick process for neutralizing residual $H_2O_2$ from a contact lens which has been in contact with an aqueous solution of $H_2O_2$, comprising:

(A) immersing the lens in an aqueous solution which contains dissolved therein

(1) agents for buffering the pH of the solution to 6.5 - 8.5;

(2) one or more salts effective, with said buffering agents, to provide the solution; under a tonicity of 0.7 to 1.4%;

(3) an optional preservative; and

(4) sodium sulfite, in an amount at least stoichiometric with respect to the residual $H_2O_2$; and

(B) keeping the lens immersed in said solution for a sufficient period of time for the sodium sulfite to neutralize the $H_2O_2$ completely.

In the preferred embodiment, the lens is immersed in buffered isotonic saline solution, and a few drops of an aqueous solution of sodium sulfite and a stabilizing agent for the sodium sulfite are added to neutralize the $H_2O_2$.

Another embodiment of this invention comprises a kit including apparatus for holding and agitating the lenses immersed in treatment solutions, plus a premixed solution or tablet by which an effective amount of sodium sulfite can be added to the treatment solution to neutralize residual $H_2O_2$.

As indicated, the present invention effectively and quickly neutralizes residual $H_2O_2$ from a hard or soft contact lens. This invention is directed at $H_2O_2$ which is on the lens surface and $H_2O_2$ which is within the lens, i.e. which has penetrated into the matrix of a polymeric hydrophilic ("soft") contact lens. Hydrophilic lens materials are well known to practitioners in this field; one example is polyhydroxyethylmethacrylate. Residual amounts of $H_2O_2$ will generally comprise a total of less than about 200 ppm of $H_2O_2$, which can nonetheless irritate the eyes.

The invention is most advantageously carried out as part of a comprehensive regimen for cleaning the contact lens. The regimen is preferably practices with the aid of a device of the type disclosed and claimed in U.S. Patent No. 3,623,492, the disclosure of which is hereby incorporated herein by reference. One such device, known as a "Hydra-Mat II", includes two perforated baskets for holding a pair of lenses inside a container which can hold enough of a liquid treatment solution to immerse the lenses. The baskets are connected by a rod to gears inside the lid which closes the container. Twisting the lid rotates the baskets about the axis of the rod, thereby washing the lenses with the treatment solution.

The lens to be disinfected is first removed from the eye and then cleaned, preferably by gentle rubbing between the fingers with a commercial lens cleaning solution for 15 - 20 seconds, or by other appropriate methods for cleaning. This step, while not an essential part of the invention, helps loosen soil from the lens surface. The loosened soil and the cleaning solution are then rinsed off of the lens with a stream of commercial buffered isotonic saline rinsing solution. Alternatively, the dirt and cleaner are washed off by placing the lens into a basket of the Hydra-Mat II, adding enough of the rinsing solution (7 ml) into the container of the Hydra-Mat II to immerse the lens, closing the lid over the container, and then turning the lid to wash the lens through the solution.

The lens is next immersed in an aqueous solution of $H_2O_2$ to disinfect the lens. Immersion is preferably carried out in the Hydra-Mat II (after the rinsing solution has been discarded). Enough of the $H_2O_2$ solution is placed in the container to immerse the lens in the basket, and the lid is closed and turned to rotate the lens within the solution. A 3 wt. % solution of $H_2O_2$ is acceptable, in which case the lens should remain immersed in the solution for 5 to 10 minutes. $H_2O_2$ concentrations up to about 6 wt. % and as low as 0.25 wt. % are acceptable, provided that the time period for which the lens is immersed in the solution is longer for weaker solutions. A satisfactory disinfecting period for any given concentration on $H_2O_2$ can be determined by observing the degree of disinfection of a sample contaminated lens which is exposed to 3 wt. % $H_2O_2$ for 10 minutes, and observing how much time is needed to achieve the same disinfection in another, equally contaminated lens in solution having an $H_2O_2$ concentration other than 3 wt. %. For instance, the lens should be held for about 6 hours in a 0.25 wt. % $H_2O_2$ solution, for about 3 hours in a 0.5 wt. % $H_2O_2$ solution, or for about 2 hours in a 1 wt. % $H_2O_2$ solution.

Following disinfection of the lens in the $H_2O_2$ solution, the lens is removed from the $H_2O_2$ solution. The $H_2O_2$ solution is discarded. The lens is then immersed in a buffered isotonic saline storage solution. There steps can be performed by literally picking up the lens from the solution and placing it into the next solution. In the Hydra-Mat II the lens can be removed from the $H_2O_2$ solution without having to touch the lens; the lid is removed with the lens still in its basket, and the container is emptied and refilled with the storage solution.

The storage solution can be one of several rinsing solutions currently available commercially, such as the one sold under the same "Soft Mate" by Barnes-Hind/Hydrocurve. The solution is buffered to a pH of 6.5 to 8.5, and preferably 7.0 to 7.4, with a buffering system such as a mixture of sodium borate and boric acid, or a sodium phosphate or sodium bicarbonate buffer. The solution should also have a sufficient tonicity to be essentially isotonic with the fluids of the eye, i.e. having a tonicity of 0.7 to 1.4% NaCl equivalents. Those familiar with this art will recognize that this range corresponds to 200 to 400 milliosmol (mOs) per kg of solution. To this end it can contain 0.3 to 1.4 wt. % of sodium chloride and/or other physiologically acceptable salts known in the ophthalmic field such as chlorides of potassium, magnesium, or calcium. The solution may also include an effective amoung of a preservative such as thimerosal, potassium sorbate, sorbic acid, chlorhexidine, methyl or propyl paraben, chlorbutanol, benzalkonium chloride, or phenyl mercuric acetate or nitrate. The most preferred buffered system is boric acid/sodium borate, which advantageously combines the functions of buffering and aiding preservation.

The rinsing solution described in the preceding paragraph has at best a slow neutralizing effect on $H_2O_2$ absorbed in or on the lens. Accordingly, the present invention includes the step of adding to the solution an amount of sodium sulfite ($Na_2SO_3$) effective to neutralize the residual $H_2O_2$. The amount added must be at least

0142642

stoichiometric with respect to the total amount of $H_2O_2$ that is absorbed in or on the lens, at the time that the lens is placed into the saline rinsing solution. Amounts up to about 10 - 100 times stoichiometric or up to about 1000 ppm of sodium sulfite per lens are acceptable.

In the preferred embodiment, the sodium sulfite is added as 3 to 5 drops of an aqueous neutralizing solution containing 0.1 to 15 wt. % of sodium sulfite. Preferably, the sodium sulfite concentration is 0.5 - 5 wt. %, and more preferably it is 2 - 3 wt. %.

A solution of sodium sulfite would be expected to be chemically unstable, and therefore impractical as a neutralizing agent. The solution would need a shelf life of months (if not years) as the bottles in which it would be sold to the public passed from the manufacturer, to store shelves, to the home of the consumer, where the solution would be opened to atmospheric oxygen repeatedly during use. Thus, in a noteworthy aspect of the present invention, the neutralizing solution also contains dissolved therein a stabilizing agent effective to prevent premature oxidation of the sodium sulfite before the neutralizing solution is added to the lens solution, and which is not injurious to the lens or to the eye. The disodium salt EDTA is a preferred stabilizing agent; other satisfactory agents include glucose, sodium lauryl sulfate, and propyl gallate. The agent can comprise about 0.005 - 0.20 wt. % of the neutralizing solution, preferably about 0.01 - 0.10 wt. %, or up to about 10% by weight of the sodium sulfite.

Sodium sulfite solutions so stabilized have been found to retain over half their peroxide-neutralizing activity when air was bubbled through the solution for 144 consecutive hours; this test presents relatively severe conditions. In another test which simulated actual use of the stabilized solution, a vial of the solution was opened daily and 3-5 drops were removed with a dropper. After a month, the peroxide-neutralizing activity was still about 90%.

The solution of sodium sulfite also advantageously contains a preservative to prevent growth of microorganisms which could be introduced during repeated opening of the solution container. The most preferred preservative is a sodium borate/boric acid buffer that is effective to control the pH of the solution to about 9, such as about 1 wt. % of this buffer. This system is effective to meet the Antimicrobial Preservative Effectiveness Test (USP No 20).

In an alternate embodiment, the sodium sulfite is added as a tablet (or several tablets) supplying sufficient sodium sulfite to neutralize the residual $H_2O_2$. The tablet can contain 0.1 - 99 wt % sodium sulfite, plus inert binders used in making the tablet according to conventional techniques.

After the sodium sulfite is added to the storage solution, the lens is preferably agitated within the solution, such as by turning the top of the Hydra-Mat II to spin the lens through the solution. Neutralization is generally complete in about 5 - 10 minites. The rapidity of the neutralization is an unexpected and advantageous feature of the invention, especially compared to neutralization with like amounts of other neutralizing agents. The rapidity is especially advantageous to wearers of extended-wear contact lenses, who frequently do not have a pair of spectacles to use while they wait for their lenses to be cleaned and neutralized.

The neutralization reaction converts the $H_2O_2$ to water, and the sodium sulfite is converted to sodium sulfate in solution which is inert to the lens. The lens can be reinserted into the eye immediately, stored in the resulting solution for several hours or overnight, or it can be removed and immersed in fresh storage solution.

Another embodiment of this invention comprises a kit for disinfecting lenses with $H_2O_2$ and for neutralizing the residual $H_2O_2$ on the lenses. The kit comprises a means for washing the lens, and a source of sodium sulfite. The kit can optionally include either or both of an aqueous solution of $H_2O_2$, and an aqueous, buffered, isotonic saline solution.

The lens washer means is advantageously of the type having

a container open at its top for receiving lens washing fluid and a lens case agitator;

a lidlike member removable mounted on the top end of the container and open at both ends and having a transverse partition intermediate its ends; said partition having an aperture extending therethrough;

a lens case agitator pivotally mounted in said aperture in the partition and extending into said container and having depending lens case supporting means and an upper end extending above the partition and having a spur gear thereon;

a knoblike member pivotally received in the upper end of the lidlike member and having finger grasp means for rotating the same and internal gear teeth therein;

0142642

planetary gear means pivotally mounted on said transverse top portion of the lidlike member and interposed between said spur gear on the agitator and said internal gear teeth in the knoblike member for imparting rotation to said agitator member whereby a lens carried in said lens case is washed in the liquid in the container; or the equivalent thereof.

The sodium sulfite source preferably comprises a vial of an aqueous solution of 2 to 3 wt. % (preferably 2.5 wt. %) sodium sulfite, 0.5 to 3.0 wt. % (preferably 1 wt. %) sodium borate, and 0.005 to 3.0 wt. % (preferably 1 wt. %) disodium EDTA salt.

The saline solution can be a commercial buffered saline rinsing solution. The solution should have a pH of 7.0 to 7.4, and buffering agents such as sodium borate/boric acid to maintain the pH in that range. The solution also contains e.g. sodium chloride, to provide a tonicity of 0.7 to 1.4 %, plus small but effective amounts of a stabilizing agent, preferably disodium EDTA, and of a preservative.

The $H_2O_2$ solution has a concentration of 1 to 6 wt. % and preferably about 3 wt. %. The $H_2O_2$ solutions used herein advantageously include up to 0.05 wt. % of a stabilizer which does not react with or discolor the lens. A satisfactory stabilizer is a mixture of sodium stannate and sodium pyrophosphate, in a ratio of about 60:40 to 40:60 by weight.

The user removes the lens from the eye, carries out the rubbing and rinsing steps described above, and encloses the lens in the perforated holding basket of the washing device. The user then places about 7 ml of 3 wt. % $H_2O_2$ aqueous solution into the container of the washing device, and closes the lid so that the lens stays immersed in the $H_2O_2$ for about 5 to 10 minutes. The kit ought to include instructions informing the user how much of a given concentration of $H_2O_2$ solution to use, and how long to keep the lens in the solution. Of course, times other than 5-10 minutes are used with concentrations other than 3 wt. %. A mark or line can be provided on the side of the container to indicate the level that 7 ml of solution reaches. Next, the user removes the $H_2O_2$ solution and replaces it with the saline solution buffered to pH 7.0 - 7.4. Into this solution the user adds 3 to 5 drops of the sodium sulfite solution; the top of the device can be twisted to help disperse the sodium sulfite in the solution. The lens is stored in this solution for 5 to about 10 minutes. At the end of this time, the total $H_2O_2$ content of the lens and the ambient solution is less than 40 ppm, and more advantageously less than 10 ppm. The lens at this point can be worn without irritation to the eye.

The invention is described in the following Example:

EXAMPLE 1

One Hydrocurve II (55% water-bufilcon A 45%) lens was put in each of the two baskets of the Hydra-Mat II. The container was filled with 7.0 ml of 3 wt. % of $H_2O_2$. The lenses were immersed in the 3 wt. % $H_2O_2$ solution and the lid was turned for 15-20 seconds to agitate the lenses in the solution. After 10 minutes, the baskets were removed from the 3 wt. % $H_2O_2$ solution which was discarded. The container and the lid with the baskets were rinsed thoroughly with a saline solution containing 0.75 wt. % sodium chloride, 0.01 wt. % disodium salt of EDTA, 0.368 wt. % potassium sorbate, 0.1% boric acid, with a pH of 7.0 and tonicity of 0.9 (sodium chloride equivalent). The container was filled with 7.0 ml of the saline solution mentioned above and 4 drops of a neutralizing solution containing 2.5 wt. % sodium sulfite, 0.01% disodium salt of EDTA and 1.0% borate with a pH of 9.2 were added. The lenses were immersed in the combined saline-neutralizing solution and the baskets were agitated for 15-20 seconds. After 5 minutes, the total amount of the residual $H_2O_2$ in the solution and lenses was found to be less than 20 mg (less than 3 ppm at 7.0 ml).

It will be recognized that all components of the solutions used herein should be non-irritating to the eye (except for the $H_2O_2$ itself, of course) and should be inert to the lens. Standard tests for determining non-irritability to the eye, and inertness to the lens, are known to those skilled in this field.

## CLAIMS

1.   A process for neutralizing $H_2O_2$ absorbed in a hard or soft contact lens, comprising

(a) immersing the lens in an aqueous solution which container dissolved therein

(1) on or more buffering agents to buffer the pH of the solution to a value of 6.5 to 8.5;

(2) one or more salts effective with said buffering agents to provide the solution with a tonicity of 0.7 to 1.4%; and

(3) sodium sulfite, in an amount at least stoichiometric with respect to the amount of $H_2O_2$ to be neutralized; and

(b) keeping the lens immersed in said solution for at least enough time to permit the sodium sulfite to neutralize all the $H_2O_2$.

2.   The process of claim 1 wherein step (a) comprises initially immersing the lens in a storage solution containing components (1) and (2), and then adding to said storage solution a neutralizing composition containing component (3).

3.   The process of claim 2 wherein said neutralizing composition comprises an aqueous neutralizing solution having a sodium sulfite concentration of 0.1 to 15 wt. % and containing a stabilizing agent in an amount up to about 10% by weight of said sodium sulfite.

4.   The process of claim 3 wherein the stabilizing agent is the disodium salt of EDTA, glucose, sodium lauryl sulfate, or propyl gallate.

5. The process of any of claims 2 to 4 wherein the sodium sulfite concentration in said neutralizing solution is about 2 to 3 wt. %.

6. The process of any of claims 2 to 5 wherein the neutralizing composition comprises a water-soluble tablet of sodium sulfite.

7. The process of any of claims 2 to 6 wherein said storage solution also contains up to 1.0 wt. % of a preservative.

8. The process of any of claims 1 to 7 wherein the buffering agent is a mixture of boric acid and sodium borate.

9. The process of any of claims 1 to 8 wherein said one or more salts is sodium chloride.

10. A process according to any of claims 2 to 9 wherein the lens is immersed in the combined storage and neutralizing solutions until the $H_2O_2$ content of the lens and the combined solutions is less than 40 ppm.

11. The process of claim 10 wherein the pH of the storage solution prior to addition of the neutralizing solution is 7.0 to 7.4; the neutralizing solution contains 0.5 to 5 wt. % sodium sulfite; and the stabilizing agent is the disodium salt of EDTA.

12. A process for disinfecting a hard or soft contact lens, comprising

(i) immersing the lens in an aqueous solution of 0.25 to 6.0 wt. % $H_2O_2$ for a period of time effective to disinfect the lens;

(ii) then removing the lens from the $H_2O_2$ solution; and

(iii) neutralizing residual $H_2O_2$ in the lens by carrying out the steps of any of claims 1 to 11.

13. A kit for neutralizing residual $H_2O_2$ absorbed to contact lens, including means for washing the lens, and a source of sodium sulfite, wherein the source is (1) an aqueous solution containing 0.5 to 5 wt. % sodium sulfite and a stabilizing agent in an amount up to 10% by weight of the sodium sulfite, or (2) a tablet of sodium sulfite.

14. A kit according to claim 13 wherein said means for washing the lens comprises a container open at its top for receiving lens washing fluid and a lens case agitator;

a lidlike member removably mounted on the top end of the container and open at both ends and having a transverse partition intermediate its ends;

said partition having an aperture extending therethrough;

a lens case agitator pivotally mounted in said aperture in the partition and extending into said container and having depending lens case supporting means and an upper end extending above the partition and having a spur gear thereon;

0142642

a knoblike member pivotally received in the upper end of the lidlike member and having finger grasp means for rotating the same and internal gear teeth therein;

planetary gear means pivotally mounted on said transverse top portion of the lidlike member and interposed between said spur gear on the agitator and said internal gear teeth in the knoblike member for imparting rotation to said agitator member whereby a lens carried in said lens case is washed in the liquid in the container.

15. A kit according to claim 13 or 14 further comprising an aqueous, physiologically acceptable saline, buffered solution having a pH of 7.0 to 7.4 and a tonicity of 200 to 400 milliosmol/kg.

16. A kit according to claim 13 or 14 wherein the washing means is an aqueous solution containing 0.25 to 6 wt. % $H_2O_2$.

## EUROPEAN SEARCH REPORT

European Patent
Office

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | FR-A-2 269 968 (INTERNATIONAL OPTICAL CORP.) * Page 1, lines 14-18, 27-36; page 2, lines 1-12; claims 1-5 * | 1-16 | A 61 L 2/18 A 01 N 59/00 G 02 C 13/00 |
| | --- | | |
| D,Y | FR-A-2 247 327 (FLOW PHARMACEUTICALS) * Claim 3; pages 11-13 * | 1-12, 15,16 | |
| | --- | | |
| D,Y | US-A-3 623 492 (D.G. FRANTZ) * Claim 1 * | 13-14 | |
| | --- | | |
| A | GB-A-1 601 430 (CONTACT LENSES MANUFACTURING) * Claim 3 * | 1 | |
| | --- | | TECHNICAL FIELDS SEARCHED (Int. Cl.4) |
| A | FR-A-2 424 311 (SENJU PHARMACEUTICAL) * Claim 1 * | 1 | |
| | ----- | | A 61 L G 02 C |

The present search report has been drawn up for all claims

| Place of search THE HAGUE | Date of completion of the search 28-11-1984 | Examiner PELTRE CHR. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82